# EUROPEAN PATENT SPECIFICATION

(11) **EP 3 170 443 B1**
(45) Date of publication and mention of the grant of the patent: **02.01.2019**
(21) Application number: 16811272.0
(22) Date of filing: 14.03.2016
(51) Int. Cl.: A61B 1/04, G02B 23/24, H01L 27/14

(54) **IMAGING MODULE, ENDOSCOPE SYSTEM, AND METHOD FOR MANUFACTURING IMAGING MODULE**
BILDGEBUNGSMODUL, ENDOSKOPSYSTEM UND VERFAHREN ZUR HERSTELLUNG EINES BILDGEBUNGSMODULS
MODULE D'IMAGERIE, SYSTÈME D'ENDOSCOPE ET PROCÉDÉ DE FABRICATION DE MODULE D'IMAGERIE

(30) Priority: 16.06.2015 JP 2015121278
(43) Date of publication of application: 24.05.2017
(73) Proprietor: Olympus Corporation, Tokyo 192-8507 (JP)
(72) Inventor: SHIMIZU, Toshiyuki, Tokyo 192-8507 (JP); MOTOHARA, Hiroyuki, Tokyo 192-8507 (JP); FUJII, Toshiyuki, Tokyo 192-8507 (JP); ISHIKAWA, Shinya, Tokyo 192-8507 (JP)
(74) Representative: Gunzelmann, Rainer
(86) International application number: PCT/JP2016/058006
(87) International publication number: WO 2016/203797

(56) References cited:
- JP-A- 2010 050 260
- JP-A- 2014 108 282
- JP-A- 2014 204 275
- JP-A- 2015 066 300
- US-A1- 2014 284 751

## Description

### Field

The present invention relates to an imaging module provided at a distal end of an insertion section of an endoscope configured to be inserted into a subject to image the inside of the subject. The present invention also relates to an endoscope system and a method for manufacturing the imaging module.

### Background

In a medical field and an industrial field, endoscope apparatuses have been widely used for various examinations. The endoscope apparatuses includes a widely-used medical endoscope apparatus which is configured such that an elongated flexible insertion section having a distal end provided with an image sensor can be inserted into a body cavity of a subject such as a patient to obtain an in-vivo image of the body cavity without incision of the subject, and a treatment tool can be further protruded from the distal end of the insertion section, if necessary, to provide treatment. The documents JP2014-108282A and JP2015066300 refer to endoscopes.

An imaging unit is fitted into such a distal end of the insertion section of the endoscope apparatus, and the imaging unit includes an image sensor, and a circuit board mounted with electronic components, such as a capacitor and an IC chip, constituting a drive circuit for the image sensor. In such an imaging unit, a connecting portion between the image sensor and the circuit board is filled with an underfill material to increase reliability of the connecting portion. Various technologies are proposed for the underfill material (e.g., see Patent Literatures 1 to 3).

### Citation List

### Patent Literature

Patent Literature 1: JP 2009-182155 A
Patent Literature 2: JP H10-270477 A
Patent Literature 3: JP 2004-214344 A

### Summary

### Technical Problem

In Patent Literature 1, at an edge of a connection surface on a lower surface of a package substrate on which electronic components are mounted, a cutout portion is provided to prevent wetting of the underfill material to an electronic component mounting surface on an upper surface of the package substrate. However, in Patent Literature 1, a main substrate larger than the package substrate is used, and a fillet of the underfill material is formed on a side surface of the package substrate, so that conversion for which reduction in size is desired is not immediately allowed.

In Patent Literature 2, a wiring substrate is provided with a through-hole to inject an underfill material into a connecting portion between the wiring substrate and a semiconductor package, through the through-hole. However, in Patent Literature 2, through-holes are provided at a center or predetermined positions of the wiring substrate, which makes wiring difficult if the substrate is reduced in size.

In Patent Literature 3, an imaging device is configured such that an image sensor having electrodes at a peripheral edge of a sensor unit (light receiving unit), and a substrate having wiring formed at a peripheral edge of a through-hole to be connected to the electrodes of the image sensor, are connected so as to face each other. In the imaging device, resin reservoirs for communicating with the through-hole are formed at four corners of the through-hole to prevent an underfill material filled in a connecting portion from flowing out to the sensor unit. However, in Patent Literature 3, the substrate larger than the image sensor is used, and a fillet of the underfill material is formed on a side surface of the image sensor, so that conversion for which reduction in size is desired is not immediately allowed.

The present invention has been made in view of the foregoing, and an object of the invention is to provide a small-size imaging module which makes it possible to increase reliability of a connecting portion, and to provide an endoscope system and a method for manufacturing the imaging module.

### Solution to Problem

In order to solve the above described problem and achieve the object, an imaging module according to the invention includes: a chip size package having an image sensor that has a light receiving unit on a front side of the image sensor, the chip size package having a plurality of connection lands on a back side of the image sensor; a circuit board having a plurality of connection electrodes being electrically and mechanically connected to the plurality of connection lands of the chip size package through bumps; and an underfill material filled into a connecting portion between the chip size package and the circuit board. The circuit board and the underfill material are provided within a projection plane on which the chip size package is projected in an optical axis direction of the image sensor. The circuit board has a cutout portion on a side surface thereof orthogonal to a connection surface of the circuit board with the chip size package such that the cutout portion is open to at least the connection surface.

In the imaging module according to the invention, the cutout portion penetrates from the connection surface on a front side of the circuit board to a back side of the circuit board.

In the imaging module according to the invention, the projection plane of the chip size package in the optical axis direction has a rectangular shape, and the plurality of connection electrodes of the circuit board is disposed away from one side of the circuit board where the cutout portion is disposed on the back side of the image sensor.

In the imaging module according to the invention, the circuit board has a rectangular shape on a projection plane on which the circuit board is projected in the optical axis direction, and the cutout portion is provided at four corners of the rectangular shape of the circuit board.

In the imaging module according to the invention, the circuit board has a solder mask layer on the connection surface with the chip size package, and the cutout portion is provided on a side surface of the solder mask layer.

An endoscope system according to the invention includes an insertion section having, on a distal end thereof, the imaging module.

A method for manufacturing the imaging module according to the invention includes: a connection step of collectively connecting a plurality of bumps on a back side of a chip size package with a plurality of connection electrodes on a circuit board; and a sealing step of inserting a tip end of a nozzle for injecting an underfill material into a cutout portion provided on part of a side surface orthogonal to a connection surface of the circuit board with the chip size package such that the cutout portion is open to the connection surface, thereby filling the underfill material into a gap in a connecting portion between the chip size package and the circuit board. Advantageous Effects of Invention

According to the present invention, it is possible to increase reliability of the connecting portion as well as to achieve a small-size imaging module.

### Brief Description of Drawings

FIG. 1 is a schematic view illustrating an overall configuration of an endoscope system according to a first embodiment of the present invention.
FIG. 2A is a side view of an imaging module disposed at a distal end portion of the endoscope illustrated in FIG. 1 (before filling an underfill material).
FIG. 2B is a diagram of a connection surface (lower surface) of a circuit board used for the imaging module of FIG. 2A.
FIG. 3A is a diagram illustrating filling an underfill material into a connecting portion of a conventional imaging module.
FIG. 3B is a side view of the conventional imaging module (after filling the underfill material).
FIG. 3C is a diagram illustrating filling the underfill material into a connecting portion of the imaging module according to the first embodiment of the present invention.
FIG. 3D is a side view of the imaging module according to the first embodiment of the present invention (after filling the underfill material).
FIG. 4 is a side view of an imaging module according to a first modification of the first embodiment of the present invention (before filling the underfill material).
FIG. 5A is a side view of an imaging module according to a second modification of the first embodiment of the present invention (before filling the underfill material).
FIG. 5B is a diagram of a connection surface (lower surface) of a circuit board used for the imaging module according to the second modification of the first embodiment of the present invention.
FIG. 5C is a diagram illustrating filling the underfill material into a connecting portion of the imaging module according to the second modification of the first embodiment of the present invention.
FIG. 5D is a side view of the imaging module according to the second modification of the first embodiment of the present invention (after filling the underfill material).
FIG. 6A is a side view of an imaging module according to a second embodiment of the present invention (before filling an underfill material).
FIG. 6B is a top view of a second circuit board used for the imaging module according to the second embodiment of the present invention.
FIG. 6C is a side view of the imaging module according to the second embodiment of the present invention (after filling the underfill material).
FIG. 7 is a side view of an imaging module according to a first modification of the second embodiment of the present invention.
FIG. 8A is a side view of an imaging module according to a second modification of the second embodiment of the present invention (before filling an underfill material).
FIG. 8B is a diagram illustrating filling the underfill material into a connecting portion of the imaging module according to the second modification of the second embodiment of the present invention.
FIG. 8C is a side view of the imaging module according to the second modification of the second embodiment of the present invention (after filling the underfill material). Description of Embodiments

An endoscope system having an imaging module will be described below as modes for carrying out the present invention (hereinafter, referred to as "embodiment(s)"). The present invention is not limited to the embodiments. The reference signs are used to designate the same elements throughout the drawings. The drawings are schematically illustrated, and relationships between the thicknesses and the widths, the ratios of members may be different from those of actual ones. The dimensions or ratios of the members may be different between the drawings.

### (First Embodiment)

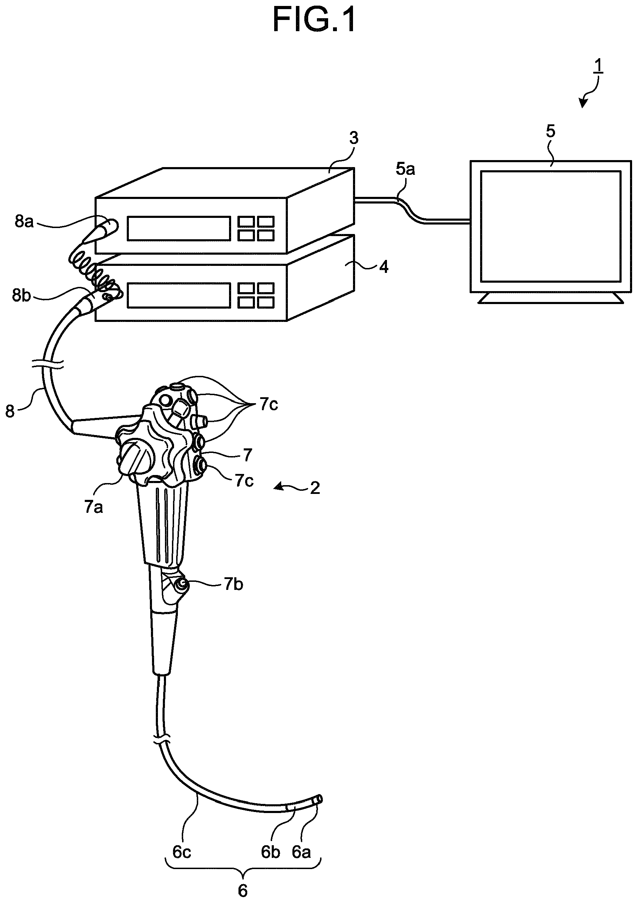

FIG. 1 is a schematic view illustrating an overall configuration of an endoscope system according to a first embodiment of the present invention. As illustrated in FIG. 1, an endoscope system 1 according to the first embodiment includes an endoscope 2 introduced into a subject, imaging inside a body of the subject, and generating an image signal representing an inside of the subject, an information processing device 3 performing predetermined image processing on the image signal captured by the endoscope 2, and controlling each element of the endoscope system 1, a light source device 4 generating illumination light of the endoscope 2, and a display device 5 displaying the image signal as an image after the image processing performed by the information processing device 3.

The endoscope 2 includes an insertion section 6 inserted into the subject, an operating unit 7 positioned at a proximal end of the insertion section 6 to be grasped by an operator, and a flexible universal cord 8 extending from the operating unit 7.

The insertion section 6 includes an illumination fiber (light guide cable), an electric cable, an optical fiber, and the like. The insertion section 6 has a distal end portion 6a having a built-in imaging unit described later, a bending section 6b including a plurality of bending pieces freely bent, and a flexible tube portion 6c provided on a proximal end side of the bending section 6b and having flexibility. The distal end portion 6a is provided with an illumination unit for illuminating the inside of the subject through an illumination lens, an observation unit for imaging inside the subject, an opening for communicating with a treatment tool channel, and an air/water feeding nozzle (not illustrated).

The operating unit 7 has a bending knob 7a for bending the bending section 6b vertically and horizontally, a treatment tool insertion portion 7b for inserting a treatment tool such as biopsy forceps or a laser scalpel into a body cavity of the subject, and a plurality of switch units 7c for operating peripheral devices such as the information processing device 3, the light source device 4, an air feeding device, a water feeding device, and a gas feeding device. The treatment tool is inserted from the treatment tool insertion portion 7b and is exposed from the opening at a distal end of the insertion section 6 through the treatment tool channel provided inside the endoscope.

The universal cord 8 includes an illumination fiber, a cable, and the like. The universal cord 8 is branched at a proximal end to have one end being a connector 8a, and the other end being a connector 8b. The connector 8a can be removably mounted to the information processing device 3. The connector 8b can be removably mounted to the light source device 4. The universal cord 8 transmits the illumination light emitted from the light source device 4 to the distal end portion 6a, through the connector 8b and the illumination fiber. The universal cord 8 transmits an image signal captured by an imaging module described later to the information processing device 3, through the cable and the connector 8a.

The information processing device 3 performs predetermined image processing on the image signal output from the connector 8a, and controls the whole endoscope system 1.

The light source device 4 includes a light source for emitting light, a condenser lens, and the like. Under the control of the information processing device 3, the light source device 4 emits light from the light source, and supplies the light, as illumination light for illuminating the inside of the subject as an object, to the endoscope 2 through the connector 8b and the illumination fiber of the universal cord 8.

The display device 5 includes a liquid crystal or organic electro luminescence (EL) display or the like. The display device 5 displays, through a video cable 5a, various information including the image subjected to the predetermined image processing by the information processing device 3. Thus, the operator can operate the endoscope 2, while viewing an image (in-vivo image) displayed on the display device 5, and a desired position in the subject can be observed and characteristics thereof can be determined.

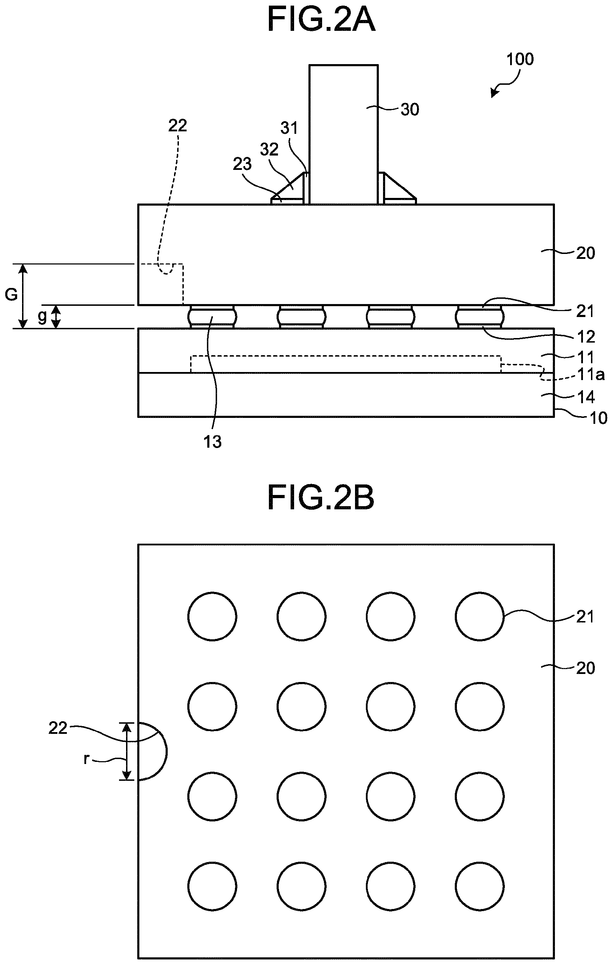

Next, the imaging module used for the endoscope system 1 will be described in detail. FIG. 2A is a side view of the imaging module disposed at the distal end portion of the endoscope illustrated in FIG. 1 (before filling an underfill material). FIG. 2B is a diagram of a connection surface (lower surface) of a circuit board used for the imaging module of FIG. 2A.

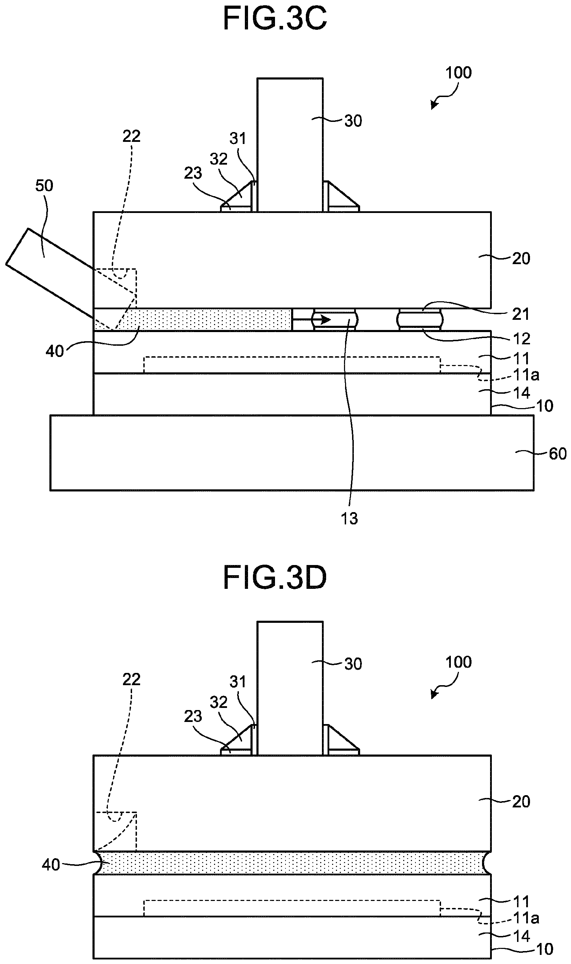

An imaging module 100 includes a chip size package 10 having an image sensor 11 which has a light receiving unit 11a on a front side of the image sensor 11 and having a plurality of connection lands 12 on a back side of the image sensor 11, a first circuit board 20 having a plurality of connection electrodes 21 which is electrically and mechanically connected to the connection lands 12 of the chip size package 10 through bumps 13, a second circuit board 30 disposed perpendicular to the first circuit board 20, and an underfill material 40 filled into a connecting portion between the chip size package 10 and the first circuit board 20 (see FIG. 3D).

Each of the first circuit board 20 and the second circuit board 30 has a rectangular plate shape, and the first circuit board 20 and the second circuit board 30 are provided within a projection plane on which the chip size package 10 is projected in an optical axis direction of the image sensor 11 when the elements of the imaging module 100 are connected to one another.

The image sensor 11 has thereon the light receiving unit 11a, such as a CMOS. The light receiving unit 11a is connected to the connection lands 12 on the back side, via through-wiring (not illustrated) formed by through-silicon via (TSV) or the like. The bumps 13 of solder are formed on the connection lands 12. A cover glass 14 for protecting the light receiving unit 11a is bonded to the surface of the image sensor 11.

The first circuit board 20 has the plate shape in which wirings are layered through an insulation layer. The first circuit board 20 includes a ceramic substrate, an epoxy glass substrate, a glass substrate, a silicon substrate, or the like. The connection electrodes 21 are formed at positions corresponding to the connection lands 12, on a connection surface of the first circuit board 20 with the chip size package 10, and a connection electrode 23 is formed on a back side of the connection surface. The connection electrodes 21 are electrically and mechanically connected to the connection lands 12 through the bumps 13. A cutout portion 22 which is open to the connection surface is formed in a side surface orthogonal to the connection surface of the first circuit board 20 with the chip size package 10.

The second circuit board 30 has the plate shape in which wirings are layered through an insulation layer. The second circuit board 30 includes a ceramic substrate, an epoxy glass substrate, a glass substrate, a silicon substrate, or the like. The second circuit board 30 has one end at which a connection electrode 31 is formed, and which is connected to the connection electrode 23 of the first circuit board 20 with solder 32. Connection between the first circuit board 20 and the second circuit board 30 is performed so that after an adhesive is applied to a predetermined position of the first circuit board 20, the second circuit board 30 is temporarily perpendicularly (T-shape) fixed to the back side of the first circuit board 20, and then the connection electrode 23 and the connection electrode 31 are connected with solder 32. Note that, although not illustrated in FIG. 2A, a cable and electronic components are connected to the second circuit board 30. Note that the electronic components may be mounted on the back side of the first circuit board 20, or may be built in the first circuit board 20 and the second circuit board 30. The mounted cable and electronic components are preferably provided within the projection plane on which the chip size package 10 is projected in the optical axis direction of the image sensor 11.

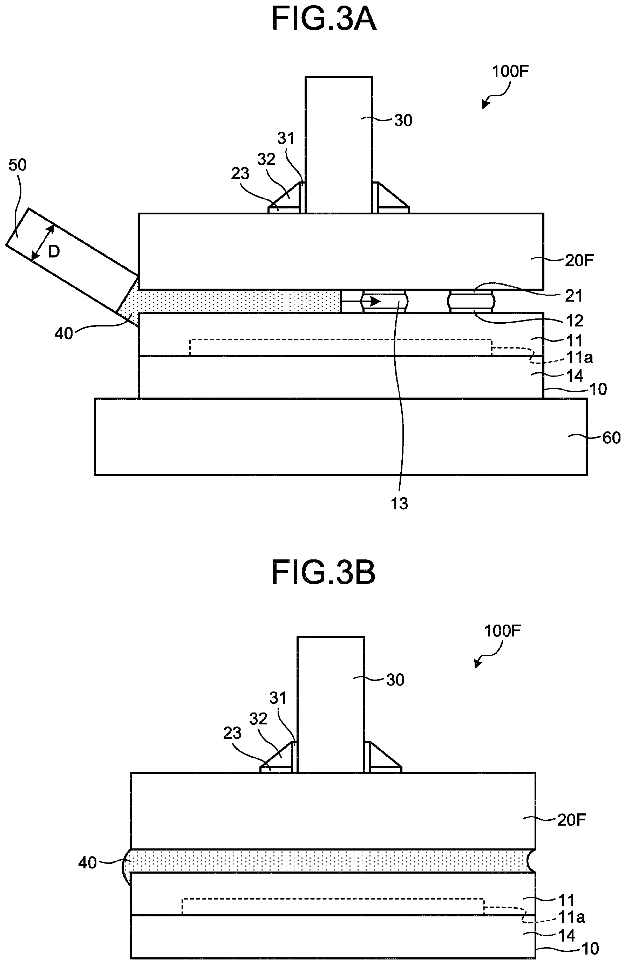

Next, filling the underfill material into the connecting portion of the imaging module according to the first embodiment will be described. Filling the underfill material into the connecting portion of the imaging module is performed by mounting the imaging module 100 in which the underfill material is not filled, on a hot plate 60 heated to approximately 60°C. FIG. 3A is a diagram illustrating filling the underfill material into a connecting portion of a conventional imaging module. FIG. 3B is a side view of the conventional imaging module (after filling the underfill material). FIG. 3C is a diagram illustrating filling the underfill material into the connecting portion of the imaging module according to the first embodiment of the present invention. FIG. 3D is a side view of the imaging module according to the first embodiment of the present invention (after filling the underfill material).

In recent years, the insertion section 6 of the endoscope 2 has been reduced in diameter to reduce a load on a specimen, and thus the imaging module 100 employs the chip size package 10 with one side having a length of approximately 1 mm to 5 mm. In the imaging module 100 using the chip size package 10 of this size, a gap between the chip size package 10 and the first circuit board 20 has a length g (see FIG. 2A) of approximately 100 µm. Since a nozzle 50 for filling the underfill material 40 has a tip diameter of 100 µm to 150 µm, the tip of the nozzle 50 cannot be inserted into a gap between the chip size package 10 and a first circuit board 20F, and the underfill material 40 is filled from a side surface of the gap, in a conventional imaging module 100F illustrated in FIG. 3A. When the underfill material 40 is filled from the side surface, the underfill material 40 not filled into the gap remains on the side surface of the chip size package 10, and thus, the imaging module 100F is increased in diameter.

In the imaging module 100 according to the first embodiment, at an end of the side surface orthogonal to the connection surface of the first circuit board 20, at least the cutout portion 22 cut out to be opened on the connection surface is formed, and the tip of the nozzle 50 is inserted into the cutout portion 22 to inject the underfill material 40 into the gap (see FIG. 3C). Since the tip of the nozzle 50 is located within the projection plane on which the chip size package 10 is projected in the optical axis direction of the image sensor 11 to fill the underfill material 40, the underfill material 40 is prevented from leaking to a side surface of the chip size package 10, which makes it possible to achieve a small-diameter imaging module 100 (see FIG. 3D). In order to fill a necessary and sufficient amount of the underfill material 40, the underfill material 40 is preferably filled, while enlarging and monitoring a portion around the cutout portion 22 for injection and a side opposed to the cutout portion 22, with two fields of view. The underfill material 40 filled in the gap is heated to approximately 120°C to 150°C, and cured.

The cutout portion 22 preferably has a sufficient size to insert the tip of the nozzle 50, and it is preferable that a diameter r of the cutout portion 22 is not less than nozzle tip diameter D + 10 µm, and a distance G from a bottom surface of the cutout portion 22 to the connection surface of the chip size package 10 is not less than nozzle tip diameter D + 10 µm. From the viewpoint of strength and packaging density, an upper limit of the diameter r of the cutout portion 22 is 20% or less of a length of one side of the first circuit board 20, preferably 10% or less. The cutout portion 22 preferably has a semicircular columnar shape, from the viewpoint of easiness in formation, but is not limited to this shape, and may have a rectangular or triangular columnar shape (tapered end surface of the first circuit board 20). The cutout portion 22 is formed at a center of one side of the first circuit board 20, but may be formed at a corner of the first circuit board 20, or a plurality of the cutout portions 22 may be provided.

In the first embodiment, the cutout portion 22 for inserting the tip of the nozzle 50 is provided on the side surface orthogonal to the connection surface of the first circuit board 20 to fill the underfill material 40 into the gap between the chip size package 10 and the first circuit board 20, through the cutout portion 22, and thus, the underfill material 40 is prevented from leaking outside the projection plane of the chip size package 10, that is, the underfill material 40 can be provided within the projection plane on which the chip size package 10 is projected in the optical axis direction of the image sensor 11, which makes it possible to achieve a small-diameter imaging module 100. Since the underfill material 40 is filled into the gap between the chip size package 10 and the first circuit board 20, reliability of the connecting portion can be increased.

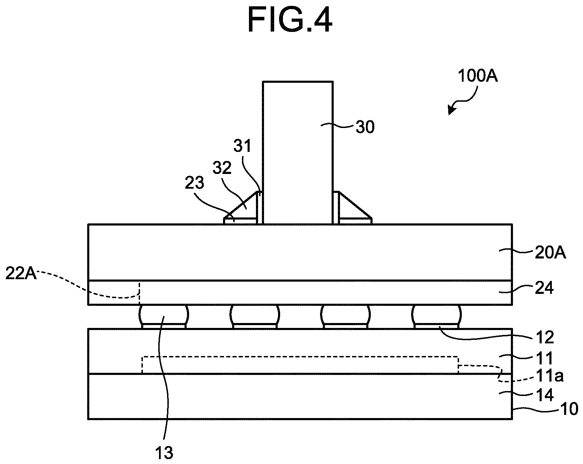

When a solder mask layer is formed on the connection surface of the first circuit board, the cutout portion may be formed in the solder mask layer. FIG. 4 is a side view of an imaging module according to a first modification of the first embodiment of the present invention (before filling the underfill material).

In an imaging module 100A according to the first modification of the first embodiment of the present invention, a solder mask layer 24 is formed on a connection surface of a first circuit board 20A. A cutout portion 22A is formed in the solder mask layer 24. The cutout portion 22A may have a semicircular columnar shape, as in the first embodiment, but the solder mask layer 24 may not be formed on one side of the first circuit board 20A so that a portion without the solder mask layer 24 is used as the cutout portion.

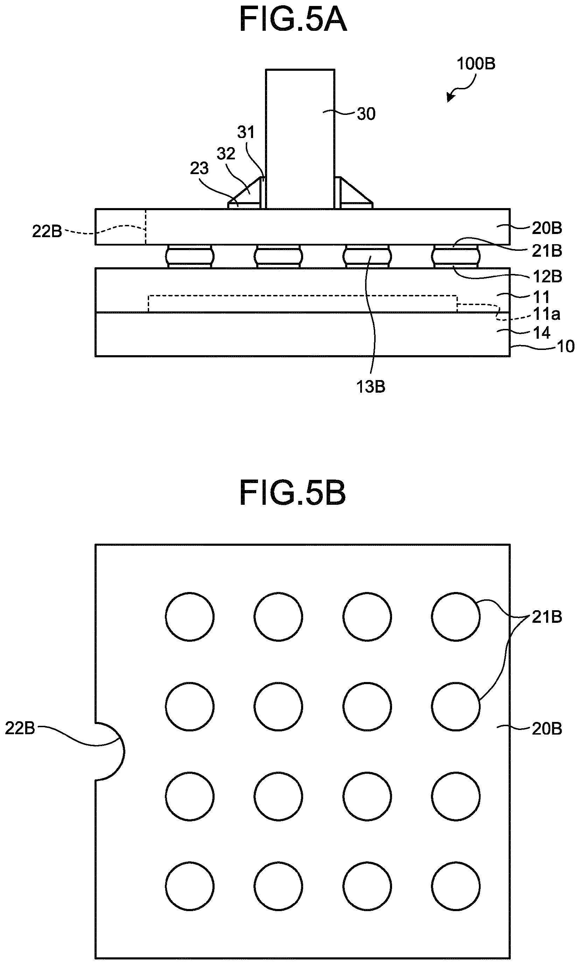

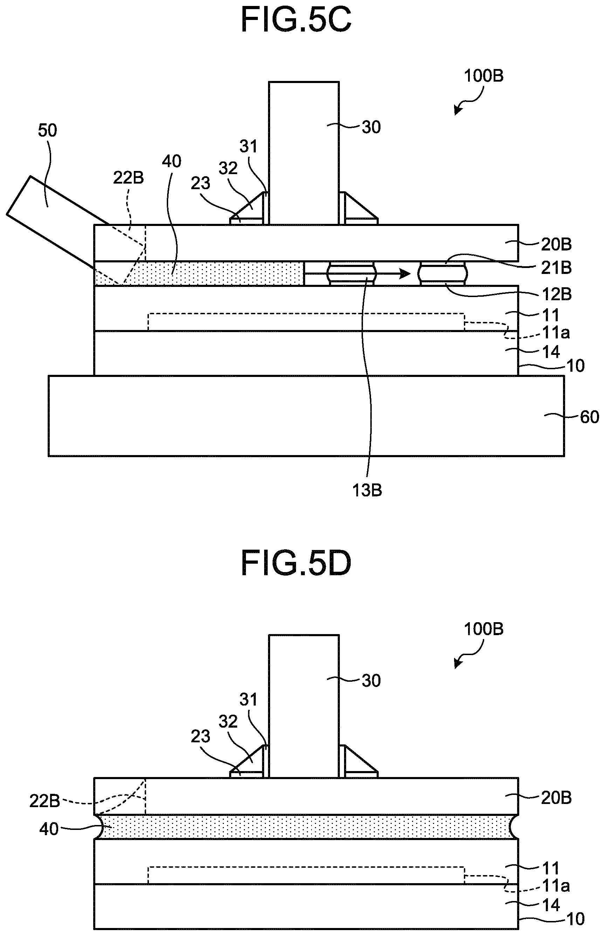

Depending on a thickness of the first circuit board, the cutout portion may be formed to penetrate from the connection surface to the back side of the chip size package. FIG. 5A is a side view of an imaging module according to a second modification of the first embodiment of the present invention (before filling the underfill material). FIG. 5B is a diagram of a connection surface (lower surface) of a circuit board used for the imaging module according to the second modification of the first embodiment of the present invention. FIG. 5C is a diagram illustrating filling the underfill material into a connecting portion of the imaging module according to the second modification of the first embodiment of the present invention. FIG. 5D is a side view of the imaging module according to the second modification of the first embodiment of the present invention (after filling the underfill material).

In an imaging module 100B according to the second modification of the first embodiment of the present invention, a cutout portion 22B penetrates from a connection surface on a front side of a first circuit board 20B with the chip size package 10 to a back side of the first circuit board 20B. When the first circuit board 20B has a thin thickness, and a through-hole can be readily formed by drilling or the like, the cutout portion 22B can be formed to penetrate the first circuit board 20B. Connection electrodes 21B on the connection surface of the first circuit board 20B with the chip size package 10, are disposed away from one side on which the cutout portion 22B is disposed, as illustrated in FIG. 5B. Connection lands 12B connected to the connection electrodes 21B through bumps 13B are also formed corresponding to the connection electrodes 21B.

Filling the underfill material 40 is performed by inserting the tip of the nozzle 50 into the cutout portion 22B, as illustrated in FIG. 5C. The cutout portion 22B has a shape and size similar to those in the first embodiment. In order to prevent leaking of the underfill material 40 toward the side surface of the chip size package 10, and the back side of the first circuit board 20B, the underfill material 40 is preferably filled, while enlarging and monitoring a portion around the cutout portion 22B for injection and a side opposed to the cutout portion 22B, with two fields of view.

In the imaging module 100B according to the second modification of the first embodiment of the present invention, when an increased amount of the underfill material 40 is filled, the underfill material 40 has a fillet shape in the cutout portion 22B, as illustrated in FIG. 5D, but the underfill material 40 does not protrude outside the projection plane on which the chip size package 10 is projected in the optical axis direction of the image sensor 11, which makes it possible to achieve a small-diameter imaging module 100B. In the imaging module 100B, since the cutout portion 22B is formed to penetrate the first circuit board 20B, a direction of the imaging module 100B can be readily determined on the basis of a position of the cutout portion 22B, and handling of the imaging module 100B is facilitated in an assembling process. In the imaging module 100B, since the connection electrodes 21B are disposed away from the one side on which the cutout portion 22B is disposed, the cutout portion 22B can be readily formed.

### (Second Embodiment)

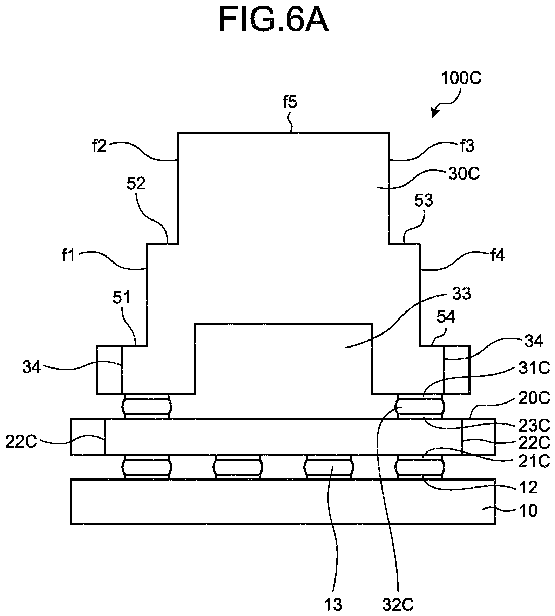

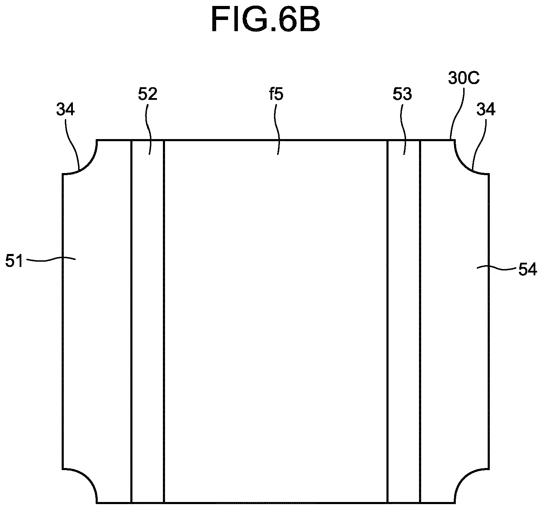

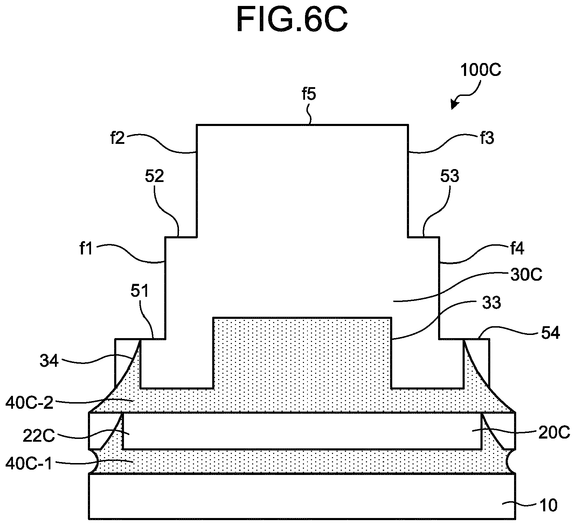

In a second embodiment, a deformed circuit board is employed as a second circuit board. FIG. 6A is a side view of an imaging module according to the second embodiment of the present invention (before filling an underfill material). FIG. 6B is a top view of the second circuit board used for the imaging module according to the second embodiment of the present invention. FIG. 6C is a side view of the imaging module according to the second embodiment of the present invention (after filling the underfill material).

A first circuit board 20C has a rectangular plate shape in which wirings are layered through an insulation layer, and the first circuit board 20C is provided within the projection plane on which the chip size package 10 is projected in the optical axis direction of the image sensor 11 when elements of an imaging module 100C are connected to one another. Connection electrodes 21C are formed at positions corresponding to the connection lands 12, on a connection surface of the first circuit board 20C with the chip size package 10, and connection electrodes 23C are formed on a back side of the connection surface. The connection electrodes 21C are electrically and mechanically connected to the connection lands 12 through the bumps 13. At four corners of the first circuit board 20C, cutout portions 22C are formed to penetrate from the connection surface to the back side of the first circuit board 20C.

A second circuit board 30C has a horizontally symmetrical deformed shape in which wirings are layered through an insulation layer, and the second circuit board 30C is provided within the projection plane on which the chip size package 10 is projected in the optical axis direction of the image sensor 11 when the elements of the imaging module 100C are connected to one another. The second circuit board 30C may employ a molded interconnect device (MID) substrate having three-dimensional wiring formed by injection molding, in addition to a substrate similar to that in the first embodiment. The second circuit board 30C has right and left stepped portions 51, 52, 53, and 54. On a bottom surface of the second circuit board 30C, connection electrodes 31C and a recessed portion 33 are formed. The connection electrodes 31C are connected to the connection electrodes 23C of the first circuit board 20C through solders 32C, and the recessed portion 33 penetrates back and forth. Electronic components (not illustrated) are mounted, in a space formed by the first circuit board 20C and the recessed portion 33. At four corners of the second circuit board 30C, cutout portions 34 are formed to penetrate from a connection surface of the second circuit board 30C with the first circuit board 20C, to the stepped portion 51 or the stepped portion 54. In the second circuit board 30C, cable connection lands (not illustrated) are formed on a surface f1 between the stepped portion 51 and the stepped portion 52, a surface f2 between the stepped portion 52 and an upper surface f5, a surface f3 between the upper surface f5 and the stepped portion 53, and a surface f4 between the stepped portion 53 and the stepped portion 54, and cables are connected to the lands.

In the imaging module 100C, underfill materials 40C-1 and 40C-2 are filled into a connecting portion between the chip size package 10 and the first circuit board 20C, and a connecting portion between the first circuit board 20C and the second circuit board 30C, respectively.

The underfill material 40C-2 is filled into the connecting portion between the first circuit board 20C and the second circuit board 30C while the tip of the nozzle is inserted into the recessed portion 33 of the second circuit board 30C such that the tip of the nozzle 50 is located within a projection plane on which the first circuit board 20C is projected in the optical axis direction. Filling the underfill material 40C-2 into the recessed portion 33 is preferably performed, while enlarging and monitoring a portion of the recessed portion 33 near an injection side, and a portion of the recessed portion 33 opposed to the recessed portion 33 on the injection side, with two fields of view. After the underfill material 40C-2 is filled in the recessed portion 33, the nozzle 50 is moved to a cutout portion 22C adjacent to the recessed portion 33, and the underfill material 40C-1 is filled into the connecting portion between the chip size package 10 and the first circuit board 20C. The underfill material 40C-1 is filled into the connecting portion between the chip size package 10 and the first circuit board 20C while the tip of the nozzle 50 is inserted into the cutout portion 22C such that the tip of the nozzle 50 is located within the projection plane on which the chip size package 10 is projected in the optical axis direction of the image sensor 11. Filling the underfill material 40C-1 is preferably performed, while enlarging and monitoring a portion around the cutout portion 22C for injection and the cutout portion 22C opposed to the cutout portion 22C, with two fields of view. Thus, the underfill material 40C-1 is prevented from leaking to a side surface of the chip size package 10.

In the imaging module 100C according to the second embodiment of the present invention, the underfill materials 40C-1 and 40C-2 have a fillet shape in the cutout portions 22C and 34, respectively, as illustrated in FIG. 6C, but the underfill materials 40C-1 and 40C-2 do not protrude outside the projection plane on which the chip size package 10 is projected in the optical axis direction of the image sensor 11, which makes it possible to achieve a small-diameter imaging module 100C. Since the cutout portions 22C and 34 are formed at four corners of the first circuit board 20C and the second circuit board 30C, even if θ displacement occurs, upon connection between the first circuit board 20C and the second circuit board 30C, or upon connection between the chip size package 10 and the first circuit board 20C, the imaging module 100C can be inhibited from being increased in diameter.

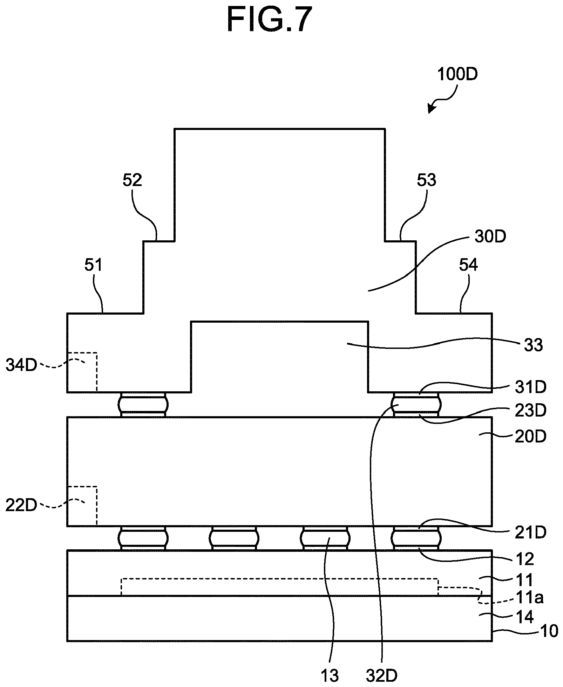

In the second embodiment, the cutout portions are formed to penetrate the first circuit board and the second circuit board, but the cutout portions may be formed not to penetrate the first circuit board and the second circuit board. FIG. 7 is a side view of an imaging module according to a first modification of the second embodiment of the present invention.

In an imaging module 100D, a first circuit board 20D has a side surface orthogonal to a connection surface with the chip size package 10, and a cutout portion 22D which is open to the connection surface is formed on the side surface. The cutout portion 22D is formed in one side surface of the first circuit board 20D, but may be formed on each side surface or at four corners thereof, as long as the cutout portion 22D has a sufficient size to insert the tip of the nozzle. A second circuit board 30D has a side surface orthogonal to a connection surface with the first circuit board 20D, and a cutout portion 34D which is open to the connection surface is formed on the side surface. The cutout portion 34D is formed in one side surface of the second circuit board 30D, but may be formed in each side surface or at four corners thereof, as long as the cutout portion 34D has a sufficient size to insert the tip of the nozzle.

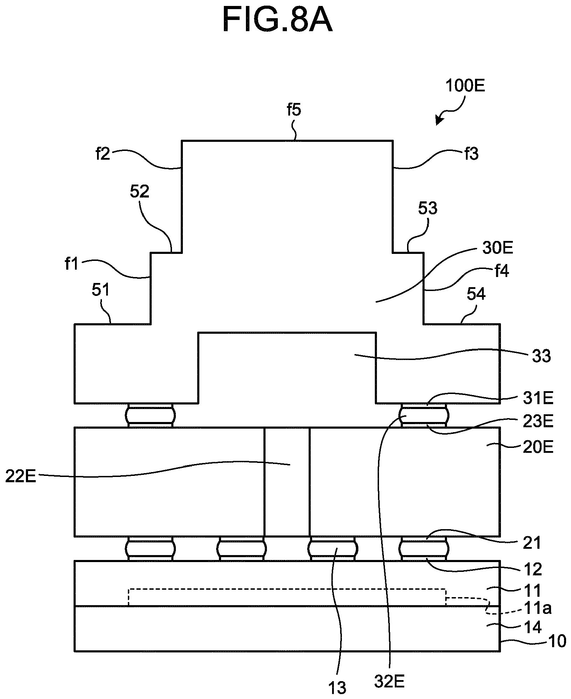

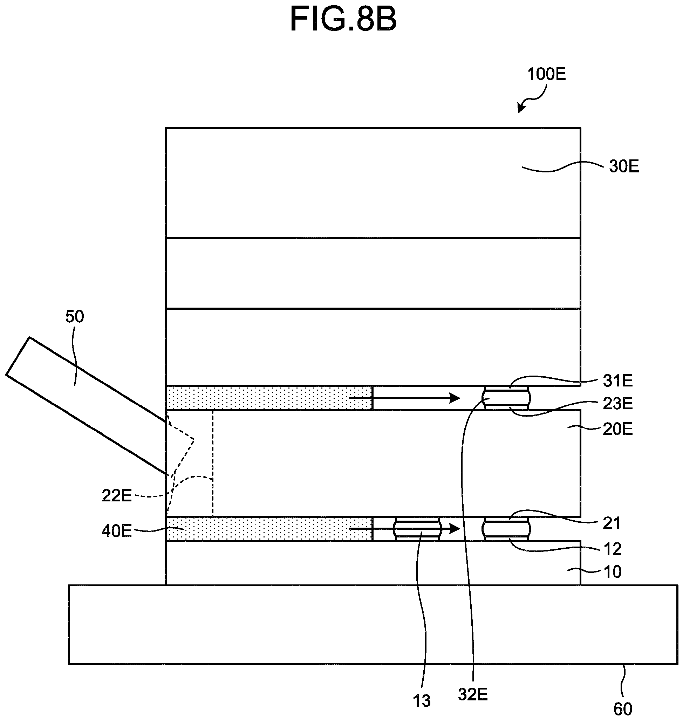

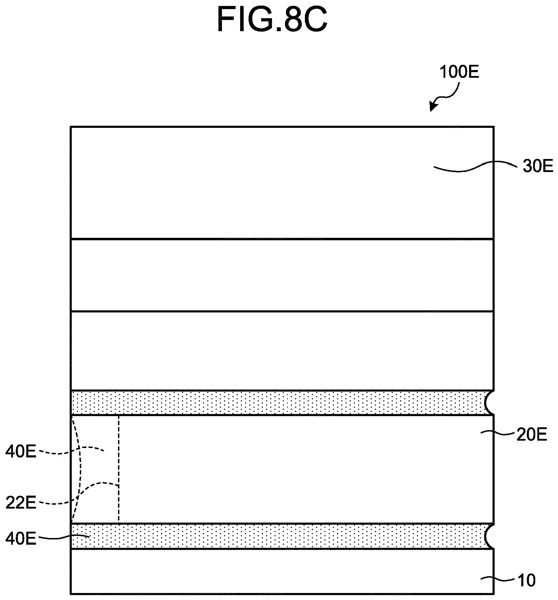

A cutout portion penetrating from the connection surface with the chip size package to a connection surface with the second circuit board may be formed in a side surface of the first circuit board so that the underfill material is filled into a connecting portion between the chip size package and the first circuit board, and a connecting portion between the first circuit board and the second circuit board, through the cutout portion. FIG. 8A is a side view of an imaging module according to a second modification of the second embodiment of the present invention (before filling an underfill material). FIG. 8B is a diagram illustrating filling the underfill material into a connecting portion of the imaging module according to the second modification of the second embodiment of the present invention. FIG. 8C is a side view of the imaging module according to the second modification of the second embodiment of the present invention (after filling the underfill material).

In an imaging module 100E, a cutout portion 22E penetrating from a connection surface with the chip size package 10 to a connection surface with a second circuit board 30E is formed in a side surface of a first circuit board 20E. Filling underfill material 40E into a connecting portion between the chip size package 10 and the first circuit board 20E, and a connecting portion between the first circuit board 20E and the second circuit board 30E is performed, while the tip of the nozzle is inserted into the cutout portion 22E formed in the first circuit board 20E, and the tip of the nozzle 50 is positioned in the chip size package 10 projected on a projection plane in the optical axis direction, as illustrated in FIG. 8B. In order to efficiently fill the underfill material 40E, the tip of the nozzle 50 may be vertically moved in the cutout portion 22E. Filling the underfill material 40E is preferably performed, while enlarging and monitoring a portion around the cutout portion 22E and a side opposed to the cutout portion 22E, with two fields of view. Thus, the underfill material 40E is prevented from leaking to a side surface of the chip size package 10.

In the imaging module 100E according to the second modification of the second embodiment of the present invention, the underfill material 40E does not protrude outside the projection plane on which the chip size package 10 is projected in the optical axis direction of the image sensor 11, as illustrated in FIG. 8C, which makes it possible to achieve a small-diameter imaging module 100E.

### Industrial Applicability

An imaging module, an endoscope system, and a method for manufacturing the imaging module according to the present invention are useful for an endoscope system requiring a high quality image and a small diameter of a distal end portion.

### Reference Signs List

1 ENDOSCOPE SYSTEM
2 ENDOSCOPE
3 INFORMATION PROCESSING DEVICE
4 LIGHT SOURCE DEVICE
5 DISPLAY DEVICE
6 INSERTION SECTION
6a DISTAL END PORTION
6b BENDING SECTION
6c FLEXIBLE TUBE PORTION
7 OPERATING UNIT
7a BENDING KNOB
7b TREATMENT TOOL INSERTION PORTION
7c SWITCH UNIT
8 UNIVERSAL CORD
8a, 8b CONNECTOR
10 CHIP SIZE PACKAGE
11 IMAGE SENSOR
11a LIGHT RECEIVING UNIT
12, 12B CONNECTION LAND
13, 13B BUMP
14 COVER GLASS
20, 20A, 20B, 20C, 20F FIRST CIRCUIT BOARD
21, 21B, 21C, 21D, 23, 23C, 23D, 23E, 31, 31C, 31D, 31E CONNECTION ELECTRODE
22, 22A, 22B, 22C, 22D CUTOUT PORTION
24 SOLDER MASK LAYER
30, 30C SECOND CIRCUIT BOARD
32, 32D, 32E SOLDER
33 RECESSED PORTION
40, 40C-1, 40C-2, 40E UNDERFILL MATERIAL
50 NOZZLE
51, 52, 53, 54 STEPPED PORTION
60 HOT PLATE
100, 100A, 100B, 100C, 100D, 100E, 100F IMAGING MODULE

## Claims

1. An imaging module (100) comprising:
a chip size package (10) having an image sensor (11) that has a light receiving unit (11a) on a front side of the image sensor, the chip size package having a plurality of connection lands (12) on a back side of the image sensor;
a circuit board (20) having a plurality of connection electrodes (21) being electrically and mechanically connected to the plurality of connection lands of the chip size package through bumps (13); and
an underfill material (40) filled into a gap between the chip size package and the circuit board, wherein
the circuit board and the underfill material are provided within a projection plane on which the chip size package is projected in an optical axis direction of the image sensor, and **characterised in that**
the circuit board has a cutout portion (22) on a side surface thereof orthogonal to a connection surface of the circuit board with the chip size package such that the cutout portion is open to at least the connection surface.

2. The imaging module according to claim 1, wherein
the cutout portion penetrates from the connection surface on a front side of the circuit board to a back side of the circuit board.

3. The imaging module according to claim 1 or 2, wherein
the projection plane of the chip size package in the optical axis direction has a rectangular shape, and
the plurality of connection electrodes of the circuit board is disposed away from one side of the circuit board where the cutout portion is disposed on the back side of the image sensor.

4. The imaging module according to claim 1 or 2, wherein
the circuit board has a rectangular shape on a projection plane on which the circuit board is projected in the optical axis direction, and
the cutout portion is provided at a corner of the circuit board.

5. The imaging module according to claim 1, wherein
the circuit board has a solder mask layer (24) on the connection surface with the chip size package, and
the cutout portion is provided on a side surface of the solder mask layer.

6. An endoscope system comprising an insertion section having, on a distal end (6a) thereof, the imaging module according to any one of claims 1 to 5.

7. A method for manufacturing the imaging module according to any one of claims 1 to 5, the method comprising:
a connection step of collectively connecting a plurality of bumps on a back side of a chip size package with a plurality of connection electrodes on a circuit board; and
a sealing step of inserting a tip end of a nozzle for injecting an underfill material into a cutout portion provided on part of a side surface orthogonal to a connection surface of the circuit board with the chip size package such that the cutout portion is open to the connection surface, thereby filling the underfill material into a gap in a connecting portion between the chip size package and the circuit board.

## Patentansprüche

1. Bildgebungsmodul (100), das umfasst:
ein Chip Size Package (10) mit einem Bildsensor (11), der eine Lichtaufnahmeeinheit (11a) auf einer Vorderseite des Bildsensors aufweist, wobei das Chip Size Package eine Mehrzahl von Anschlussstegen (12) auf einer Rückseite des Bildsensors aufweist;
eine Leiterplatte (20) mit einer Mehrzahl von Anschlusselektroden (21), die über Bondhügel (13) elektrisch und mechanisch mit der Mehrzahl von Anschlussstegen des Chip Size Package verbunden sind; und
ein Unterfüllungsmaterial (40), das in eine Lücke zwischen dem Chip Size Package und der Leiterplatte gefüllt ist, wobei
die Leiterplatte und das Unterfüllungsmaterial innerhalb einer Projektionsebene bereitgestellt sind, auf der das Chip Size Package in einer Optische-Achse-Richtung des Bildsensors projiziert ist, und **dadurch gekennzeichnet, dass**
die Leiterplatte einen ausgeschnittenen Abschnitt (22) auf einer Seitenfläche davon orthogonal zu einer Anschlussfläche der Leiterplatte mit dem Chip Size Package aufweist, so dass der ausgeschnittene Abschnitt zu zumindest der Verbindungsfläche offen ist.

2. Bildgebungsmodul nach Anspruch 1, wobei
der ausgeschnittene Abschnitt von der Anschlussfläche auf einer Vorderseite der Leiterplatte zu einer Rückseite der Leiterplatte durchdringt.

3. Bildgebungsmodul nach Anspruch 1 oder 2, wobei
die Projektionsebene des Chip Size Package in der Optische-Achse-Richtung eine rechteckige Form aufweist, und
die Mehrzahl von Anschlusselektroden der Leiterplatte weg von einer Seite der Leiterplatte angeordnet ist, auf der der ausgeschnittene Abschnitt auf der Rückseite des Bildsensors angeordnet ist.

4. Bildgebungsmodul nach Anspruch 1 oder 2, wobei
die Leiterplatte eine rechteckige Form auf einer Projektionsebene aufweist, auf der die Leiterplatte in der Optische-Achse-Richtung projiziert ist, und
der ausgeschnittene Abschnitt an einer Ecke der Leiterplatte bereitgestellt ist.

5. Bildgebungsmodul nach Anspruch 1, wobei
die Leiterplatte eine Lötstopplackschicht (24) auf der Anschlussfläche mit dem Chip Size Package aufweist, und
der ausgeschnittene Abschnitt auf einer Seitenfläche der Lötstopplackschicht bereitgestellt ist.

6. Endoskopsystem, das einen Einsetzabschnitt mit dem Bildgebungsmodul nach einem der Ansprüche 1 bis 5 an einem distalen Ende (6a) davon umfasst.

7. Verfahren zum Herstellen des Bildgebungsmoduls nach einem der Ansprüche 1 bis 5, wobei das Verfahren umfasst:
einen Anschlussschritt des gesammelten Anschließens einer Mehrzahl von Bondhügel auf einer Rückseite eines Chip Size Package mit einer Mehrzahl von Anschlusselektroden auf einer Leiterplatte; und
einen Versiegelungsschritt des Einsetzens eines Spitzenendes einer Düse zum Injizieren eines Unterfüllungsmaterials in einen ausgeschnittenen Abschnitt, der auf einem Teil einer Seitenfläche orthogonal zu einer Anschlussfläche der Leiterplatte mit dem Chip Size Package bereitgestellt ist, so dass der ausgeschnittene Abschnitt zur Anschlussfläche offen ist, wodurch das Unterfüllungsmaterial in eine Lücke in einem Anschlussabschnitt zwischen dem Chip Size Package und der Leiterplatte gefüllt wird.

## Revendications

1. Module d'imagerie (100) comprenant :
un boîtier (10) de la taille d'une puce, ayant un capteur d'image (11) qui a une unité de réception de lumière (11a) sur un côté avant du capteur d'image, le boîtier de la taille d'une puce ayant une pluralité de pastilles de connexion (12) sur un côté arrière du capteur d'image ;
une carte de circuit (20) ayant une pluralité d'électrodes de connexion (21) et reliée électriquement et mécaniquement à la pluralité de pastilles de connexion du boîtier de la taille d'une puce par l'intermédiaire de bosses (13) ; et
un matériau de remplissage sous-jacent (40) remplissant un intervalle entre le boîtier de la taille d'une puce et la carte de circuit,
la carte de circuit et le matériau de remplissage sous-jacent étant disposés dans un plan de projection sur lequel le boîtier de la taille d'une puce est projeté dans une direction d'axe optique du capteur d'image, et **caractérisé par le fait que**
la carte de circuit a une partie de découpe (22) sur une surface latérale de celle-ci orthogonale à une surface de connexion de la carte de circuit avec le boîtier de la taille d'une puce, de telle sorte que la partie de découpe est ouverte au moins sur la surface de connexion.

2. Module d'imagerie selon la revendication 1, dans lequel
la partie de découpe pénètre de la surface de connexion sur un côté avant de la carte de circuit, jusqu'à un côté arrière de la carte de circuit.

3. Module d'imagerie selon la revendication 1 ou 2, dans lequel
le plan de projection du boîtier de la taille d'une puce dans la direction d'axe optique a une forme rectangulaire, et
les différentes électrodes de connexion de la carte de circuit sont disposées à distance d'un côté de la carte de circuit où la partie de découpe est disposée sur le côté arrière du capteur d'image.

4. Module d'imagerie selon la revendication 1 ou 2, dans lequel
la carte de circuit a une forme rectangulaire sur un plan de projection sur lequel la carte de circuit est projetée dans la direction d'axe optique, et
la partie de découpe est prévue à un coin de la carte de circuit.

5. Module d'imagerie selon la revendication 1, dans lequel
la carte de circuit a une couche de masque de soudure (24) sur la surface de connexion avec le boîtier de la taille d'une puce, et
la partie de découpe est prévue sur une surface latérale de la couche de masque de soudure.

6. Système d'endoscope comprenant une section d'insertion ayant, sur une extrémité distale (6a) de celle-ci, le module d'imagerie selon l'une quelconque des revendications 1 à 5.

7. Procédé de fabrication du module d'imagerie selon l'une quelconque des revendications 1 à 5, le procédé comprenant :
une étape de connexion, consistant à connecter collectivement plusieurs bosses sur un côté arrière d'un boîtier de la taille d'une puce, avec plusieurs électrodes de connexion sur une carte de circuit ; et
une étape de scellement, consistant à insérer une extrémité de pointe d'une buse pour injecter un matériau de remplissage sous-jacent dans une partie de découpe prévue sur une partie d'une surface latérale orthogonale à une surface de connexion de la carte de circuit avec le boîtier de la taille d'une puce, de telle sorte que la partie de découpe est ouverte sur la surface de connexion, remplissant ainsi de matériau de remplissage sous-jacent un intervalle dans une partie de connexion entre le boîtier de la taille d'une puce et la carte de circuit.
